# EUROPEAN PATENT APPLICATION

(11) **EP 1 849 458 A1**
(43) Date of publication of application: **31.10.2007**
(21) Application number: 06712207.7
(22) Date of filing: 24.01.2006
(51) Int. Cl.: A61K 9/00

(54) **METHOD OF SELECTING TITANIUM OXIDE OR PIGMENT TAKING INTO ACCOUNT ENVIRONMENT UNDER MULTIPLE LIGHT SOURCES, AND COMPOSITION THEREOF**

(30) Priority: 24.01.2005 JP 2005044702; 24.01.2005 JP 2005044703; 24.01.2005 JP 2005044704; 13.06.2005 JP 2005172076
(71) Applicant: Cosmetechno Co., Ltd., Minamiashigara-shi Kanagawa, 2500125 (JP)
(72) Inventor: KURODA, Akihiro Yokohama Office Cosmetechno Co Ltd, Yokohama-shi Kanagawa 2260011 (JP)
(74) Representative: Vossius & Partner
(86) International application number: PCT/JP2006/300991
(87) International publication number: WO 2006/078026

(57) **Abstract**

In the selection of pigment for use, the optical characteristics of pigment taking into account an environment under multiple light sources obtained when irradiating each object with visible light from two or more light sources but not obtained in the use of a single light source can be utilized in compositions, or moldings, containing pigments, such as titanium dioxide, suitable for cosmetic, etc.

## Description

### BACKGROUND OF THE INVENTION

### Technical Field

The present invention relates to a method for selecting a pigment or titanium dioxide considering an environment where multiple light sources, not a single light source, are present, as well as a compound containing the same. In particular, the present invention provides a selection method wherein the different ways in which pigments color appearance are considered and a desired pigment is selected by quantitatively understanding the color appearance, in order to select a pigment or titanium dioxide more suitable for an actual living environment where multiple light sources are present. In addition, the present invention relates to utilization of an appropriate pigment selected by the aforementioned method in a compound or molding as its ingredient.

### Prior Art

Traditionally, a measuring method that uses a single light source, such as a colorimeter or variable-angle spectrophotometer (Non-patent Literature 1), is used to determine the colors of cosmetics, coating films of paints, etc. This is partly because a complex light-source configuration makes it difficult to analyze the result, and partly because the traditional color theory is built on the assumption of a single light source and therefore a multiple light-source environment itself has not heretofore been studied sufficiently. As for light sources themselves, color temperatures and spectra are studied in details, but other aspects, such as colored light sources and relationship of multiple light sources, are little explored. One situation where multiple light sources are used is a photo studio. However, the technology employed in photo studios is to capture objects and people more beautifully, and no research has been done to analyze and select pigments using the aforementioned technology or to analyze colors under multi-colored light sources arranged in a certain layout.

Non-patent Literature 1: http://www.mcrl.co.jp/keisoku/color/color03/color-03.html#GCMS-4 (searched January 16, 2005)

### SUMMARY OF THE INVENTION

### Problems to Be Solved by the Invention

On the other hand, it is rare that light is received only from a single light source in the actual situations where we conduct our life, where almost always multiple light sources are combined to create colors and shades of objects. For example, in homes many lights for dressers and washing basins use incandescent lamps, while most ceiling lights use fluorescent lamps. With these familiar items alone, we already have two types of light sources. The inventors focused on this point and conducted extensive studies to understand the effects of using multiple light sources and ways in which such effects can be utilized in industrial applications.

### Means for Solving the Problems

After studying the subject matters diligently, the inventors found that objects would sometimes look clearly different under a single light source and multiple light sources in terms of various optical effects relating to color, and that this phenomenon could be found in various aspects of our daily life. For example, irradiating yellow light onto a film coated with a titanium dioxide makes the film look yellow when seen from the other side. However, irradiating light from a fluorescent lamp onto this other side of the film changes the color to red, while the original side directly receiving the yellow light changes to blue. This is not a trick of the eyes, but an optical phenomenon, because these color changes can be captured with a camera. This phenomenon may be behind the situation where formulations containing both clear pigments and titanium dioxides, such as foundations and other cosmetics, sometimes look unsightly because the changed color of transmitted light mixes with the color of the foundation and appears on the surface to create a visibly unattractive color. By using the present evaluation method to select a titanium dioxide resistant to such color change and then designing a desired formation based on the selected titanium dioxide, a cosmetic can be obtained that does not change color easily due to light even when a clear pigment is contained in the material.

Also, the present invention has shown to provide a more beautiful coating film on other types of formulation, such as paint. Another example is a film containing a titanium dioxide, often used as a container package for cosmetic and food material. The color of such film looks beautiful under a single light source, but may become dull and unsightly under multiple light sources. This problem can also be solved when a titanium dioxide undergoing little color change is selected beforehand under multiple light sources, because such titanium dioxide prevents dull color and improves the impression of the product.

The present invention is based on a method for selecting a pigment, wherein a pigment is selected by irradiating visible light onto a film coated with a pigment, irradiating visible light onto the created shade to the extent not erasing the shade, and then examining the color of the new shade created.

To be specific, the present invention is basically characterized by the following constitutions:
(1) A method for selecting an appropriate pigment when selecting a pigment to be used, by utilizing optical characteristics of each pigment obtained when visible light is irradiated onto a target object from two or more light sources.
(2) A method for selecting a pigment according to (1), characterized in that a pigment is selected by irradiating a first visible light onto a clear resin film coated with a coating solution in which a pigment is dispersed in a clear resin, irradiating a second visible light onto the created shade to the extent not erasing the shade, and then examining the color of the new shade created.

(3) A method for selecting a pigment according to (1) or (2), characterized in that the clear resin film coated with a pigment is constituted by uniformly coating and drying on a clear resin film a coating solution in which the pigment is dispersed in a clear resin at a content of 10 percent by weight, so that the film thickness becomes 10 µm after drying.
(4) A method for selecting a pigment according to any one of (1) to (3), characterized in that the first visible light to be irradiated onto a clear resin film coated with a coating solution in which a pigment is dispersed in a clear resin, is yellow light.

(5) A method for selecting a pigment according to any one of (1) to (4), characterized in that the second visible light is emitted from a fluorescent lamp with a color temperature of 3200K or above.
(6) A method for selecting a pigment according to any one of (1) to (5), characterized in that, when the color of the shade created by irradiating a second visible light is bluish to purplish, a pigment having less color in this range is selected.
(7) A method for selecting a pigment, characterized in that selection of a pigment according to any one of (1) to (6) relates to selection of a pigment used in various products.

(8) A method for selecting a pigment according to (7), characterized in that the pigment is an inorganic powder, organic powder, organic pigment, metal powder for surface active agent, colored pigment, pearl pigment, metal powder pigment, tar dye, or natural dye.
(9) A method for selecting a pigment according to (7) or (8), characterized in that the pigment is a titanium dioxide.

(10) A method for selecting a pigment, characterized in that the various products according to (7) are selected from among cosmetics, moldings, containers, paints, exterior wall materials, wall papers, and brochures.
(11) A method for selecting a titanium dioxide according to (9), characterized in that, when a titanium dioxide is used as a pigment, yellow light is irradiated as a first visible light onto a clear resin film coated with a coating solution in which a titanium dioxide is dispersed in a clear resin, after which a second visible light is irradiated from the side opposite to the one irradiated by the yellow light, and then a titanium dioxide that produces less reddish color is selected based on the difference in red as recognized when the transmitted yellow light is observed.

(12) A method for selecting a titanium dioxide according to (9), characterized in that, when a titanium dioxide is used as a pigment, a clear resin film is uniformly coated with a coating solution in which a titanium dioxide is dispersed in a clear resin at a content of 10 percent by weight, and then the coating solution is dried to create a film of 2 µm in thickness after drying.
(13) A method for selecting a titanium dioxide according to (9), characterized in that, when a titanium dioxide is used as a pigment, the visible light irradiated onto a clear resin film coated with a coating solution in which a titanium dioxide is dispersed in a clear resin is emitted from a fluorescent lamp with a color temperature of 3200K or above.

(14) A method for selecting a titanium dioxide according to (9), characterized in that a first visible light is irradiated onto an object coated with a coating solution in which a titanium dioxide is dispersed in a clear resin, after which a second visible light is irradiated onto the object from a different light source, and then a titanium dioxide that makes the surface condition of the object less conspicuous is selected.
(15) A method for selecting a titanium dioxide according to (9), characterized in that a first visible light is irradiated onto an object, after which a second visible light is irradiated onto the object from a different light source and the appearance of the titanium dioxide is captured with a camera, and then the inconspicuousness of the surface condition of the object is evaluated by comparing the appearance of the surface.

(16) A method for selecting a titanium dioxide according to (9), characterized in that the first visible light has a color other than red.
(17) A method for selecting a titanium dioxide according to (9), characterized in that the second visible light is light from a fluorescent lamp.
(18) A method for selecting a titanium dioxide according to (9), characterized in that the primary particle size of the titanium dioxide that makes the surface condition of the object inconspicuous is not in a range of 0.1 µm or larger and smaller than 0.4 µm, but in a range of 0.05 µm or larger and smaller than 0.1 µm, or in a range of 0.4 µm or larger and 5 µm or smaller.
(19) A method for selecting a titanium dioxide according to (9), characterized in that the object is selected from among human skin, synthetic leather and leather.

(20) A cosmetic characterized by a beautiful color that is retained even under two or more light sources, and containing an organic pigment or clear pigment as well as a pigment selected by irradiating a first visible light onto a clear resin film coated with a coating solution in which a pigment selected according to any one of (1) to (9) above is dispersed in a clear resin, and then irradiating a second visible light onto the created shade to the extent not erasing the shade, after which the color of the shade created by the second visible light is examined and a pigment having less color is selected.

(21) A cosmetic according to (14), characterized in that the total content of one or more titanium dioxides is in a range of 1 to 15 percent by weight relative to the total weight of the cosmetic, while the total content of one or more clear pigments is in a range of 0.1 to 80 percent by weight relative to the total weight of the cosmetic.
(22) A molding characterized by a coating film having a beautiful color, which is achieved by the color of the shade formed by irradiating a first visible light onto a clear resin film coated with a coating solution in which a pigment selected according to any one of (1) to (9) above is dispersed in a clear resin, and then irradiating a second visible light onto the created shade to the extent not erasing the shade.

(23) A molding characterized by use of a less bluish titanium dioxide, which is obtained by evaluating the color of the shade formed by irradiating a first visible light onto a film coated with a titanium dioxide, which is used as a pigment and selected according to any one of (1) to (9) above, and then irradiating a second visible light onto the created shade to the extent not erasing the shade.
(24) A display material showing an evaluation image of an item containing a titanium dioxide selected according to any one of (1) to (9).

### Effects of the Invention

As explained above, it is clear that the present invention provides a method for selecting a pigment or titanium dioxide more suitable for the actual environment of daily life where multiple light sources are normally present, by focusing on the different ways in which pigments color appearance under multiple light sources, compared to when only a single light is present, and by quantitatively understanding the color appearance. It is also clear that by blending an appropriate pigment obtained through the present selection method, a compound offering excellent transmitted color, shade color, appearance, etc., under multiple light sources can be achieved.

### Brief Description of the Drawings

[Fig. 1] Transmitting electron micrograph (primary particle size: 0.25 µm)
[Fig. 2] Transmitting electron micrograph (primary particle size: 0.5 µm)
[Fig. 3] Transmitting electron micrograph (primary particle size: 0.7 µm)
[Fig. 4] Transmitting electron micrograph (primary particle size: 1.0 µm)
[Fig. 5] Transmitting electron micrograph (primary particle size: 5.0 µm)

### Best Mode for Carrying Out the Invention

The following explains the present invention in details.
The present invention relates to a method for selecting color under multiple light sources, and provides multiple evaluation methods and pigment selection methods according to the applicable evaluation type and optical phenomenon. The first embodiment of the present invention is a method for selecting a pigment, wherein a pigment is selected based on the color of the shade created by irradiating visible light onto a film coated with a pigment and then irradiating visible light onto the created shade to the extent not erasing the shade. This method utilizes the optical phenomenon that, while black shade is created when a single visible light is irradiated onto a film coated with a pigment, shades of various colors are created under a multiple light-source environment where visible light is irradiated onto the original shade to the extent not erasing the shade.
Conventional color measuring systems, such as a colorimeter, use only a single light source and are therefore not capable of evaluating phenomena occurring under multiple light sources.

The second embodiment of the present invention is a method for selecting a titanium dioxide, wherein yellow light is irradiated onto a clear resin film coated with a titanium dioxide, after which visible light is irradiated from the side opposite to the one irradiated by the yellow light, and then a titanium dioxide that produces less reddish color is selected based on the difference in red as recognized when the transmitted yellow light is observed. When only yellow light is irradiated onto a film coated with a titanium dioxide and then the film is viewed from the opposite side, yellow color is seen (example of a single light source). However, when light from a fluorescent lamp is irradiated onto this side (example of multiple light sources), the color changes to red, while the original side directly receiving the yellow light changes to blue. Since titanium dioxides have a high refractive index, titanium-dioxide pigments cause light to scatter more on the surface. It is considered, therefore, that combination of this light scattering effect of a titanium dioxide with the phenomenon occurring under multiple light sources changes the color of light transmitting through the titanium dioxide.

Unlike the aforementioned two embodiments, the third embodiment of the present invention relates to how objects look. To be specific, it is a method for selecting a titanium dioxide, wherein visible light is irradiated onto an object coated with a titanium dioxide, after which visible light is irradiated onto the object from a different light source, and based on the resulting appearance of the titanium dioxide one or more titanium dioxides that would make the surface condition of the object inconspicuous are selected and blended into the target formulation. When a titanium dioxide is observed on the skin under a single light source emitting visible light, the conditions of both the titanium dioxide and skin are unclear under red light having a long wavelength. As the wavelength of the light decreases, however, it becomes possible to clearly see the conditions of the titanium dioxide coating as well as the condition of the skin. These conditions can be observed in significant details under blue light.

If the same area is irradiated simultaneously with a fluorescent lamp at a different angle (although this lamp need not be a fluorescent lamp, when the actual environment of daily life is considered it is preferable to use a fluorescent lamp in Japan or incandescent lamp in Europe as it closely reflects the actual lighting condition in each region), the skin surface condition can be observed fairly clearly under any light other than red. In particular, the surface condition can be observed with marked clarity under yellow light and green light. Especially a combination of yellow light with a fluorescent lamp is often seen in our daily life as mentioned above, and in fact the optical effects of these multiple light sources are likely having great impact on aspects of our daily life. Therefore, selecting a pigment according to this phenomenon has significance. By using an optimal pigment according to each optical phenomenon, a product offering better aesthetic appeal can be achieved.

The following explains how to get the best characteristics out of each pigment, how to evaluate these characteristics, and what the desirable pigment characteristics are, among others, according to the three evaluation methods described above. The first is the method for evaluating a pigment, wherein a pigment is selected based on the color of the shade created by irradiating visible light onto a film coated with a pigment and then irradiating visible light onto the created shade to the extent not erasing the shade. Specifically, this method relates to cosmetics characterized by containing a pigment selected on the condition of having lighter color according to the aforementioned pigment selection method and based on the observed color of shade, as well as an inorganic pigment, and having a beautiful color under multiple light sources. This method can also be utilized in display materials that are used to express degrees of attractiveness or unsightliness of coating film colors based on their shades, as well as container, resin films and various products.

Pigments conforming to the present invention have a primary particle size of 1 nm to 1 mm, where the particle has the shape of a sphere, bar, roughly sphere, spindle, sheet or any indeterminable shape, etc., and include various pigments commonly used in industrial applications.
The applicable pigments include, for example, inorganic powders, organic powders, metal salt powders for surface active agent, colored pigments, pearl pigments, metal powder pigments, tar dyes, and natural dyes. Specifically, inorganic powders include titanium dioxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, gold mica, red mica, black mica, lithia mica, silicic acid, silicic anhydride, aluminum silicate, magnesium silicate, aluminum magnesium silicate, calcium silicate, barium silicate, strontium silicate, metal salt of tungstic acid, hydroxyapatite, vermiculite, hydilite, bentonite, montmorillonite, hectorite, zeolite, ceramic powder, dicalcium phosphate, alumina, aluminum hydroxide, boron nitride, boron nitride, and silica.

Organic powders include polyamide powder, polyester powder, polyethylene powder, polypropylene powder, polystyrene powder, polyurethane powder, benzoguanamine powder, polymethyl benzoguanamine powder, polytetrafluoroethylene powder, polymethyl methacrylate powder, cellulose powder, silk powder, 12 nylon, 6 nylon or other nylon powder, polyacrylic powder, polyacrylic elastomer, styrene-acrylate copolymer, divinyl benzene-styrene copolymer, vinyl resin, urea resin, phenol resin, fluororesin, silicic resin, acrylic resin, melamine resin, epoxy resin, polycarbonate resin, microcrystal fiber powder, starch powder, and lauroyl lysine.

Metal salt powders for surface active agent (metal soaps) include zinc stearate, aluminum stearate, calcium stearate, magnesium stearate, zinc myristate, magnesium myristate, zinc cetyl phosphate, calcium cetyl phosphate, and sodium zinc cetyl phosphate.
Colored pigments include iron oxide, iron hydroxide, iron titanate and other inorganic red pigments; γ-iron oxide and other inorganic brown pigments; yellow iron oxide, yellow ocher and other inorganic yellow pigments; black iron oxide, carbon black and other inorganic black pigments; manganese violet, cobalt violet and other inorganic purple pigments; chromium hydroxide, chromium oxide, cobalt oxide, cobalt titanate and other inorganic green pigments; deep blue, navy blue and other inorganic blue pigments; laked tar dyes; laked natural dyes; and synthetic resin powders combining powders of the foregoing.

Pearl pigments include titanium-dioxide coated mica, titanium-dioxide coated mica, bismuth oxychloride, titanium-dioxide coated bismuth oxychloride, titanium-dioxide coated talc, scale foil, titanium-dioxide coated colored mica, and titanium-dioxide/iron-oxide coated mica. Metal powder pigments include aluminum powder, copper powder, and stainless powder. Tar dyes include red 3, red 104, red 106, red 201, red 202, red 204, red 205, red 220, red 226, red 227, red 228, red 230, red 401, red 505, yellow 4, yellow 5, yellow 202, yellow 203, yellow 204, yellow 401, blue 1, blue 2, blue 201, blue 404, green 3, green 201, green 204, green 205, orange 201, orange 203, orange 204, orange 206, and orange 207. Natural dyes include pigments such as carminic acid, laccaic acid, carsamine, brazilin, and crocin. Among others, titanium dioxide is most preferable as it offers high refractive index and easily scatters light.

The aforementioned pigments may be given surface treatment to add water repellency, hydrophilicity, etc. Examples of water-repellent surface treatment include methyl hydrogen polysiloxane treatment, silicone resin treatment, silicone gum treatment, acrylic silicone treatment, fluorinated silicone treatment and other organosiloxane treatments; zinc stearate treatment and other metal soap treatments; silane coupler treatment, alkyl silane treatment and other silane treatments; organic titanate treatment, organic aluminate treatment, perfluoroalkyl silane treatment, perfluoroalkyl phosphate ester treatment, perfluoropolyether treatment and other fluorine compound treatments; N-lauroyl-L-lysine treatment and other amino acid treatments; squalane treatment and other oil treatments; and alkyl acrylate treatment and other acrylic treatments; which may be used alone or one or more of the foregoing may be combined.
Examples of hydrophilic surface treatment include agar treatment, deoxyribonucleic acid treatment, lecithin treatment, polyacrylate treatment, silica treatment, alumina treatment, and zirconium treatment. These pigments may or may not be given high-dispersion treatment.
Of the aforementioned pigments, combining one or more yellow powders such as gold pearl (mica titanium), iron-doped titanium dioxide, ultrafine iron oxide, iron-containing synthetic mica, yellow colored ceramics, and yellow dye, may neutralize the blue shade with the complement color and thereby improve the visual impression more effectively. The content of each of these pigments may be determined as deemed appropriate according to the degree of yellow, color development property, object in which the pigment is used, and other characteristics.

Next, the method to produce a pigment-coated film is explained. Here, it is desirable to disperse a pigment into a resin, and then coat the pigment-dispersed resin onto a clear resin film using an applicator, etc. This resin should preferably be nitrocellulose, polyester resin or any other resin having no or minimal color. If the resin is colored, it may become difficult to determine if a given outcome is an optical effect of the pigment or effect of the resin. Also, proper dispersion cannot be achieved when a resin alone is used because of viscosity, and therefore the pigment and resin should preferably be combined with a solvent made of hexane, acetone, lower alcohol, toluene or volatile silicone, among others.

Dispersion can be achieved using a disper, medium-type wet grinder, roll mill, paint shaker, etc., but a paint shaker is preferred because its operation is easy. The film to be coated must be clear, such as a film made of polyethylene terephthalate sheet, polystyrene sheet or polypropylene sheet, among which polyethylene terephthalate sheet is most desirable because it does not deform or get eroded by solvent easily. The most preferable coating conditions are a pigment content of 10 percent by weight, and film thickness of 10 µm after the solvent has dried.

A pigment-coated film produced above is irradiated with a first visible light. If an incandescent lamp is used, the power output of the lamp should be 60 to 100 W. If the power output is less than 60 W, the light is weak and difference cannot be observed clearly. If the power output exceeds 100 W, on the other hand, heat generation makes a long-term evaluation difficult. The visible light may be white light or colored light. Using colored light, especially yellow light, in the test has the advantage of producing a clear result. Colored light can be produced with a colored lamp or by applying a filter over a reflector lamp, etc. The latter method is preferred, as it provides greater optical output.
When the film is irradiated with the first light source this way, black shade is created. Next, this shade is irradiated with a second light source. This light may be white light or colored light. However, irradiation of white light is preferred, because irradiating colored light will require a separate analysis of the effect of the colored light and consequently the data will become complex. If actual living space is to be simulated, use of a fluorescent lamp is preferred.

If a fluorescent lamp is used, the lamp should desirably have a color temperature of 3200K or above. Also, irradiation with the fluorescent lamp should be indirect so that the shade created by the first light source is not erased. There is no need for strong irradiation to the extent that the original shade is erased. This way, the black shade created by the first light source changes to a bluish to purplish color under the second light source. Such color makes the skin look very pale and is not desirable.

Next, when coating films of various pigments are evaluated using the above method, it is shown that certain pigments, such as ultrafine titanium dioxides and ultrafine zinc oxides, produce shade whose color does not change much to bluish to purplish. When a pigment whose color does not change easily is selected and used in a formulation, desired effects can be demonstrated when the formulation is used in a manner where it is affected by created shade. For example, cosmetics for daytime affected by multiple light sources include those that contain pigments to shield ultraviolet light, while cosmetics that form shade and are also affected by multiple light sources include those containing such pigments in combination with organic pigments. With these cosmetics, if an organic pigment that transmits visible light is contained even a little, multiple light sources may interact with each other in the coating film on the formulation, even when the non-organic pigment content is 1 percent by weight or more relative to the weight of the formulation. In this case, the organic pigment content should preferably be adjusted to 0.1 percent by weight or more relative to the weight of the formulation.
Examples of organic pigments include silicone resin, spherical silicone elastomer powder, polyalkyl silsesquioxane, cellulose powder, nylon, polystyrene, polyethylene, polypropylene, acrylate powder, polyethylene terephthalate, N-acylated lysine, and alginate powder, which may be used alone or one or more of the foregoing may be combined.

These powders relatively have a tendency to transmit visible light and offer excellent touch. Cosmetics obtained by using these powders are resistant to the effects of outside light and can also maintain a beautiful texture of the cosmetic-applied skin under various lighting environments. Similarly, paints and inks containing clear materials are sometimes affected by the optical effects discussed in connection with the present invention. Unlike with cosmetics where making the skin look beautiful is of prime importance, use of clear materials may be desirable with paints and inks under certain conditions and it may not be necessary to forcibly reduce the bluish to purplish color. In these circumstances, it is possible to select and use a pigment-grade of titanium dioxide of rutile type whose primary particle size is in a range of 2 to 3 µm, to enhance such color.

An example of the quantification method to be used in the above evaluation is explained. A digital camera is used to capture the color of shade under the same condition without using a flash, and the captured image is converted to color space coordinates (such as CIE L*a*b* values specified by the International Commission on Illumination) on a computer using an imaging software (such as PhotoShop manufactured by Adobe), to quantify the color of shade and use the obtained values to select a pigment that best suits the application.

Display materials according to the present invention express degrees of attractiveness or unsightliness of color of coating film, based on the color of the shade formed by irradiating visible light onto a film coated with a pigment and then irradiating visible light onto the created shade to the extent not erasing the shade. For example, the present invention can be applied to brochures, exhibition materials and computer images pertaining to cosmetics, paints, exterior wall materials and wallpapers. With these items, it is effective to show the color data not only as numerical values, but also using photographs.

Many examples of a multiple light-source environment are found in homes, such as the mixed use of incandescent lamps and fluorescent lamps for dressers and in living rooms as mentioned above. Food containers and cosmetic containers are handled in these locations. Traditionally, these containers use a titanium dioxide offering high brightness as selected by measuring the level of whiteness under a single light source. When such titanium dioxide offering high brightness (pigment-grade titanium dioxides are used in general) is evaluated using the aforementioned method, however, the shade is seen as blue and this blue shade reflects on the other side of the container, which may produce an unsightly color in some cases. Products subject to such undesirable effect are not designed by taking the actual usage of the product into consideration. Containers conforming to the present invention are designed by taking the color of shade into consideration, which ensures a beautiful appearance when the container is in use.

For the same reason explained above, resin films using a pigment selected by the present evaluation method are also useful. In particular, the present invention can be favorably used for shrink films, cover films and labeling films used on the exterior of containers.

Now, the following explains the second evaluation method, or the method for selecting a titanium dioxide wherein yellow light is irradiated onto to a film coated with a titanium dioxide, after which visible light is irradiated from the side opposite to one irradiated by the yellow light, and then a titanium dioxide that produces less reddish color is selected based on the difference in red as recognized when the transmitted yellow light is observed. Cosmetics, compositions (titanium-dioxide coated films themselves are considered compositions) whose color is little affected by transmitted color, as well as display materials that present problem of transmitted color under multiple light sources, are also explained.

Under the method for selecting a titanium dioxide explained herein, yellow light is irradiated onto to a film coated with a titanium dioxide, after which visible light is irradiated from the side opposite to one irradiated by the yellow light, and then a titanium dioxide that produces less reddish color is selected based on the difference in red as recognized when the transmitted yellow light is observed. Under the present invention, the term "titanium dioxide" refers to a titanium dioxide of rutile, anatase, bulkite, amorphous or titaniasol (titania hydroxide) type, or any mixture thereof. Any titanium dioxide may be used as long as its primary particle size is in a range of 1 nm to 100 µm. The shape of titanium dioxide may be a sphere, bar, spindle, sheet or any indeterminable shape, etc. Such titanium dioxide may be given various surface treatments, such as an inorganic treatment using silica or alumina, water-repellent treatment, or hydrophilic treatment.

As for the method used herein to produce a film coated with a titanium dioxide, a preferred method is to disperse a titanium dioxide in a resin and then coat the titanium-dioxide dispersed resin onto a clear resin film using an applicator, etc. This resin should preferably be nitrocellulose, polyester resin or any other resin having no or minimal color. If the resin is colored, it may become difficult to determine if a given outcome is an optical effect of the pigment or effect of the resin. Also, proper dispersion cannot be achieved when a resin alone is used because of viscosity, and therefore the pigment and resin should preferably be combined with a solvent made of hexane, acetone, lower alcohol, toluene or volatile silicone, among others.

Dispersion can be achieved using a disper, medium-type wet grinder, roll mill, paint shaker, etc., but a paint shaker is preferred because its operation is easy. The film to be coated must be clear, such as a film made of polyethylene terephthalate sheet, polystyrene sheet or polypropylene sheet, among which polyethylene terephthalate sheet is most desirable because it does not deform or get eroded by solvent easily. The most preferable coating conditions are a pigment content of 10 percent by weight, and film thickness of 2 µm after the solvent has dried. Although the size of coating film is not specifically limited, a size between B5 and A4 is appropriate.

The yellow light used in the present invention should preferably be produced using a yellow bulb (such as MUSI manufactured by Toshiba Lightec), or by applying a yellow filter over a xenon lamp, reflector lamp or other white light source. However, lamps that naturally have yellow hue, such as incandescent lamps and reflector lamps can also be used.
In evaluating the difference in color tone, it is preferable to use a lamp whose output is 60 to 100 W because such lamp can produce a clearer result.

The visible light used as the second light source under the present invention may or may not be colored. When the actual living environment is considered, however, it is preferable to use a fluorescent lamp in Japan or incandescent lamp in Europe as it closely reflects the actual lighting condition in each region. If a fluorescent lamp is used, its color temperature should preferably be 3200K or above, or more preferably be 5000K or above. Preferably, the second light source should be positioned on the side of the titanium-dioxide coated film opposite to the one irradiated by the yellow light (so that the second light source irradiates the back side of the coated film). As long as the back side is irradiated, the space angle can be set to a desired value. Since the color changes according to the position of the second light source, however, preferably an appropriate position should be determined. The best way is to position the second light source above the extended line of the axis of irradiation of the first light source. As for the intensity of the second light source, excessively high intensity only increases the burden on the measuring person by making the difference less clear. Accordingly, a preferred method is to adjust the output to a level at which some red color is seen when yellow light is irradiated onto the coated film. Also, using a xenon lamp, etc., as the second light source and irradiating strong light from the lamp directly over a short distance is not desirable, because it makes the film look glossy and renders the data analysis difficult.

Transmitted yellow light is observed visually or using a camera under the above conditions. When observing color, do not directly view the direction of yellow light, but tilt the top side of the film by approx. 30 degrees toward the first light source with respect to the axis of irradiated yellow light, and then observe the top portion in the area directly receiving yellow light (where the first light source is seen through the film). This area is where the color changes most under the evaluation method proposed by the present invention.
Although visual inspection is the best way to capture subtle change in light under the present invention, visual inspection lacks quantification capability and therefore use of a camera is preferred. Either a silver-salt film camera or a digital camera that uses a CCD element to electronically record images can be used. However, the latter is more preferred because the captured data can be checked on the spot and the color can be easily converted to color space coordinates using a computer.

When titanium dioxides are tested under the aforementioned method, pigment-grade titanium dioxides whose average primary particle size is in a range of 0.2 to 0.4 µm generally exhibit the strongest red, while the colors of large particle size titanium dioxides whose primary particle size is greater than those of pigment-grade titanium dioxides are generally much weaker. Ultrafine titanium dioxides, which are known to produce considerable scattering of light, have even weaker red than large particle size titanium dioxides. However, various color changes occur in other ranges due to the effect of secondary aggregation of titanium dioxide, etc., and therefore it is desirable to observe color change by creating a coating film under the actual condition (for example, under a condition where the secondary aggregate is not broken if a lot of secondary aggregate is introduced into the product). Also, the above test should preferably be conducted in a dark room where no other light but the light from the specified light source is available. Analysis under multiple light sources requires many elements to be controlled and tends to become very cumbersome. In this respect, the condition should be kept as constant as possible when examining the result. If the examination result finds that generation of red color should be avoided while maintaining sufficient concealing effect, use of a large particle size titanium dioxide is a possible option.

Cosmetics conforming to the present invention are characterized by containing one or more titanium dioxides selected by the aforementioned method for selecting a titanium dioxide, and one or more clear pigments. The problems presented by clear pigments are explained below. Traditionally, makeup products, such as foundations, did not use clear pigments much. Even if clear pigments were used, they were used only to adjust the touch. In old days (such as 15 years ago), foundations mainly consisted of sericite and other inorganic body pigments, titanium dioxide, and iron oxide. Accordingly, light was reflected on the coating film surface. Even if a titanium dioxide producing red color was used, therefore, the transmitted color did not return to the top surface from the coating film and there was no negative effect.

These reflective formulations resulted in a clearly "made up" look. However, the recent trend of cosmetics is one that emphasizes a more "natural" look. As a technique to produce a more natural look, clear pigments that transmit some light have become widely used. When a clear pigment is contained, light not only reflects on the coating film surface, but multiple scattering also occurs inside the coating film and refracted light appears on the surface.

In terms of appearance, clear pigments allow for formation of a coating film with optical depth, which makes it possible to create a texture closer to the human skin. On the other hand, however, transmitted color of titanium dioxide has become a problem in some cases because some light enters the coating film and then returns to the top surface. In particular, cosmetics of this type sometimes make the face look darkish-red during twilight hours when artificial lighting and sunlight are mixed in a complex manner. These phenomena may be partly attributable to optical characteristics (clearly they are partly caused by coming-off and wetting of the makeup). When clear pigments are used, therefore, it is preferable to use titanium dioxide materials that offer better optical characteristics under multiple light sources. In fact, sample cosmetics produced using titanium dioxides selected by the present method resulted in a significantly less degree of apparent dull complexion.

Here, clear pigments mean pigments whose whiteness drop to one-half or below when mixed in dry state with oil, and include organic pigments and inorganic compounds such as sapphire and ruby, for example. However, layered clay minerals traditionally used as inorganic body pigments are not considered clear pigments, because they have been shown not to produce the aforementioned characteristic much. Under the present invention, organic pigments can be used particularly favorably.

Organic pigments used under the present invention are pigments constituted by organic compounds. For example, organic powders of this type include polyamide powder, polyester powder, polyethylene powder, polypropylene powder, polystyrene powder, polyurethane powder, benzoguanamine powder, polymethyl benzoguanamine powder, polytetrafluoroethylene powder, polymethyl methacrylate powder, cellulose powder, silk powder, 12 nylon, 6 nylon or other nylon powder, polyacrylic powder, polyacrylic elastomer, styrene-acrylate copolymer, divinyl benzene-styrene copolymer, vinyl resin, urea resin, phenol resin, fluororesin, silicic resin, acrylic resin, melamine resin, epoxy resin, polycarbonate resin, microcrystal fiber powder, starch powder, and lauroyl lysine. In particular, silicone resin, spherical silicone elastomer powder, polyalkyl silsesquioxane, cellulose powder, nylon, polystyrene, polyethylene, polypropylene, acrylate powder, polyethylene terephthalate, N-acylated lysine and alginate powder are preferred, which may be used alone or one or more of the foregoing may be combined. These powders relatively have a tendency to transmit visible light and offer excellent touch. The metal salts of the foregoing are considered organic pigments.

Although the blending ratios of the above ingredients are not specifically limited for cosmetics conforming to the present invention, it is particularly desirable that the total content of one or more titanium dioxides selected by the aforementioned method be adjusted to a range of 1 to 1 percent by weight relative to the total weight of the cosmetic, while the total content of one or more clear pigments be adjusted to a range of 0.1 to 80 percent by weight relative to the total weight of the cosmetic.
When the respective contents are kept within these ranges, the method proposed by the present invention can be used effectively in preventing the effect of transmitted red color of titanium dioxide.

Cosmetics conforming to the present invention refer to foundations, face powders, eye shadows, nail colors, makeup bases, mascaras, filter colors and other makeup products. Among others, the present invention can be applied favorably to foundations.

In addition to the materials described above, cosmetics conforming to the present invention can also use various materials commonly used in cosmetics, such as pigments, UV absorbents, oils, pigments, surface active agents, fluorine compounds, resins, mucilaginous agents, preservatives, aromatic agents, moisture-keeping agents, salts, solvents, antioxidants, chelating agents, neutralizing agents, pH adjusting agents, insect repellents or bioactive agents. For example, pigments conforming to the present invention are those having a primary particle size of 1 nm to 1 mm, where the particle has the shape of a sphere, bar, spindle, sheet or any indeterminable shape, etc., and include various pigments commonly used in industrial applications.

Examples of pigments include inorganic powders, organic powders, metal salt powders for surface active agent, colored pigments, pearl pigments, metal powder pigments, tar dyes, and natural dyes. Specifically, inorganic powders include titanium dioxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, gold mica, red mica, black mica, lithia mica, silicic acid, silicic anhydride, aluminum silicate, magnesium silicate, aluminum magnesium silicate, calcium silicate, barium silicate, strontium silicate, metal salt of tungstic acid, hydroxyapatite, vermiculite, hydilite, bentonite, montmorillonite, hectorite, zeolite, ceramic powder, dicalcium phosphate, alumina, aluminum hydroxide, boron nitride, boron nitride, and silica.

Metal salt powders for surface active agent (metal soaps) include zinc stearate, aluminum stearate, calcium stearate, magnesium stearate, zinc myristate, magnesium myristate, zinc cetyl phosphate, calcium cetyl phosphate, and sodium zinc cetyl phosphate.
Colored pigments include iron oxide, iron hydroxide, iron titanate and other inorganic red pigments; γ-iron oxide and other inorganic brown pigments; yellow iron oxide, yellow ocher and other inorganic yellow pigments; black iron oxide, carbon black and other inorganic black pigments; manganese violet, cobalt violet and other inorganic purple pigments; chromium hydroxide, chromium oxide, cobalt oxide, cobalt titanate and other inorganic green pigments; deep blue, navy blue and other inorganic blue pigments; laked tar dyes; laked natural dyes; and synthetic resin powders combining powders of the foregoing.

Pearl pigments include titanium-dioxide coated mica, titanium-dioxide coated mica, bismuth oxychloride, titanium-dioxide coated bismuth oxychloride, titanium-dioxide coated talc, scale foil, titanium-dioxide coated colored mica, and titanium-dioxide/iron-oxide coated mica. Metal powder pigments include aluminum powder, copper powder, and stainless powder.

Tar dyes include red 3, red 104, red 106, red 201, red 202, red 204, red 205, red 220, red 226, red 227, red 228, red 230, red 401, red 505, yellow 4, yellow 5, yellow 202, yellow 203, yellow 204, yellow 401, blue 1, blue 2, blue 201, blue 404, green 3, green 201, green 204, green 205, orange 201, orange 203, orange 204, orange 206, and orange 207.

Natural dyes include pigments such as carminic acid, laccaic acid, carsamine, brazilin, and crocin. These pigments may be given surface treatment to add water repellency, hydrophilicity, etc. Examples of water-repellent surface treatment include methyl hydrogen polysiloxane treatment, silicone resin treatment, silicone gum treatment, acrylic silicone treatment, fluorinated silicone treatment and other organosiloxane treatments; zinc stearate treatment and other metal soap treatments; silane coupler treatment, alkyl silane treatment and other silane treatments; organic titanate treatment, organic aluminate treatment, perfluoroalkyl silane treatment, perfluoroalkyl phosphate ester treatment, perfluoropolyether treatment and other fluorine compound treatments; N-lauroyl-L-lysine treatment and other amino acid treatments; squalane treatment and other oil treatments; and alkyl acrylate treatment and other acrylic treatments; which may be used alone or one or more of the foregoing may be combined. Examples of hydrophilic treatment include agar treatment, deoxyribonucleic acid treatment, lecithin treatment, and polyacrylate treatment. These pigments may or may not be given high-dispersion treatment.
Each of the aforementioned pigments can also be combined with various yellow powders, as mentioned earlier, to correct blue color more effectively.

The present invention also covers display materials showing the optical characteristics, under multiple light sources, of titanium dioxides selected as explained above. These display materials include brochures, exhibition materials and computer images pertaining to cosmetics, paints, resins, exterior wall materials and wallpapers. With these items, it is effective to show the color data not only as numerical values, but also using photographs.

It is considered that the present invention can also allow these optical effects and countermeasures to be applied to resins, paints, printed matters and the like, other than cosmetics, in which a titanium dioxide is dispersed in a clear resin. Accordingly, the aforementioned selection method is also effective for compositions having a clear appearance. With these compositions, the titanium dioxide is already present in a clear resin or oil, and therefore it need not be combined with any clear pigment.

The third evaluation method is explained below.
Under this method, visible light is irradiated onto an object coated with a titanium dioxide, after which visible light is irradiated onto the object from a different light source, and based on the resulting appearance of the titanium dioxide one or more titanium dioxides that would make the surface condition of the object inconspicuous are selected and blended into a cosmetic, etc. In other words, this method relates to a method for selecting a titanium dioxide that allows for selection of a titanium dioxide more suitable for the actual condition, and consequently creates, for example, a cosmetic that creates a more beautiful look, based on an understanding of color change that occurs under multiple light sources, not under a single light source, and various products such as display materials utilizing the aforementioned effects, as well as cosmetics that make lines and irregular skin texture inconspicuous and also have an excellent exterior color.

Under the method for selecting a titanium dioxide as proposed by the present invention, visible light is irradiated onto an object coated with a titanium dioxide, after which visible light is irradiated onto the object from a different light source, and based on the resulting appearance of the titanium dioxide one or more titanium dioxides that would make the surface condition of the object inconspicuous are selected and blended into a cosmetic, etc. Titanium dioxides of rutile, anatase, bulkite, amorphous or titaniasol (titania hydroxide) type, or any mixture thereof, can be used. Any titanium dioxide may be used as long as its primary particle size is in a range of 1 nm to 50 µm. However, preferably the primary particle size should not be in a range of 0.1 µm or larger and smaller than 0.4 µm, but it should be in a range of 0.05 µm or larger and smaller than 1 µm, or in a range of 0.4 µm or larger and 5 µm or smaller.

Titanium dioxides whose primary particle size fall under these ranges are highly likely to produce relatively good results under the selection method conforming to the present invention. The shape of titanium dioxide may be a sphere, bar, spindle, sheet or any indeterminable shape, etc. Such titanium dioxide may be given various surface treatments, such as an inorganic treatment using silica, alumina or zirconium, water-repellent treatment, or hydrophilic treatment. One favorable example of the titanium dioxide used under the present invention is a large particle size titanium dioxide whose primary particle size is approx. 1 to 3 µm. When a titanium dioxide meeting the aforementioned ranges is selected and used properly, favorable effects can be achieved, such as a beautiful coated surface, little effect of multiple light sources, and a translucent look in the case of a cosmetic.

Next, under the present invention the term "visible light" refers to light whose wavelength is in a range of 400 to 800 nm. Visible light may have broad waveforms such as light emitted from incandescent lamps and xenon lamps, or they may have a bright-line spectrum such as light emitted from fluorescent lamps. Color may also be added to light using a filter, etc. Many areas of optical phenomena caused by multiple light sources are not fully understood yet, and therefore we must depend on actual phenomena such as what will happen when which combination is tested. When analyzing phenomena, it is advantageous, in terms of handling ease, to use a light source with broad waveforms and add color using a filter. If colored light is used, use of any color other than red, or especially yellow or green color, is suitable for observing the skin surface condition. If red light is used, the condition under multiple light sources becomes only slightly different from the condition under a single light source, and thus not much surface information can be revealed regarding the object.

As for the object used under the present invention, human skin is most preferred. For example, one favorable method is to apply a titanium dioxide on the arm and then irradiate visible light onto the applied area. However, since the skin condition varies significantly from one person to another and also quantifying the observed result is not feasible, synthetic leather or animal hide can also be used favorably.

Here, an object coated with a titanium dioxide is irradiated with visible light, and then visible light is irradiated onto the object from a different light source. This different light source should preferably be positioned to the rear of the location on the object irradiated with the first visible light. Even if the second light source is positioned to the front of the aforementioned location, optical effects can still be observed. However, the effect of the first light source and that of the second light source interact with each other and make it difficult to analyze the outcome. Also, it is desirable that the light quantity or distance of the second light source be adjusted to a level where the shade created by the first light source is not erased. If the light quantity is excessive, observation must be performed under strong light, which is the same as a condition in a photo studio where images are taken under multiple light sources. The phenomena covered by the present invention occur only with a light quantity enough to create shade, which represents a condition close to what we encounter in daily life. After measurements have been taken, however, it is preferable to conduct the subsequent test in a dark room to facilitate the analysis of observed phenomena.

Next, under the present invention an object coated with a titanium dioxide is irradiated with visible light, after which visible light is irradiated onto the object from a different light source, and based on the resulting appearance of the titanium dioxide one or more titanium dioxides that would make the surface condition of the object inconspicuous are selected. Basically, the most reliable way to evaluate this appearance is visual inspection. Since visual inspection cannot quantify the observed result, however, it is desirable to use a camera to capture images of the object and then compare the images to select a favorable titanium dioxide. As for the camera, a silver-salt film camera or a digital camera that uses a CCD element to electronically capture images may be used. However, the latter is more useful because the captured data can be checked instantly.

On the other hand, the present invention also relates to display materials showing evaluation images of items containing titanium dioxides selected by the aforementioned method for selecting a titanium dioxide. The display materials conforming to the present invention may be, for example, product brochures, exhibition materials, newspaper articles for press release, magazine articles, or posters, each showing an item that contains a titanium dioxide selected under multiple light sources. Such titanium-dioxide containing items include cosmetics, paints, inks, papers, coated boards and resins, among others.

Cosmetics conforming to the present invention include those that contain titanium dioxides, such as foundations, concealers, face powders, eye shadows, lipsticks, eye shadows, nail colors, filter colors, sunscreens, makeup bases, skin milks, creams, lotions, and beauty essences. Preferably the titanium dioxide content in a cosmetic should be in a range of 0.1 to 35 percent by weight relative to the weight of the cosmetic formulation. In particular, the effects of the present invention can be achieved easily with concealers that normally contain a large amount of titanium dioxide. Also, the present invention can be applied to non-cosmetic materials, such as paints, inks, resins, tiles, papers, printed matters, glass, and fibers.

In addition to the selected titanium dioxide, cosmetics conforming to the present invention can also use various materials commonly used in cosmetics, such as pigments, UV absorbents, oils, pigments, surface active agents, fluorine compounds, resins, mucilaginous agents, preservatives, aromatic agents, moisture-keeping agents, salts, solvents, antioxidants, chelating agents, neutralizing agents, pH adjusting agents, insect repellents or bioactive agents.

The present invention can be used to evaluate formulations using each of the evaluation methods explained above. For example, effects of combining a makeup base with a foundation can be evaluated using a sample prepared by applying the two ingredients in layers on clear sheets, or a sample combining sheets coated with the respective ingredients. In this type of evaluation, unique effects can be achieved when a ultrafine powder, for example, is combined. For example, when a commercial multi-layer separation type makeup base containing a ultrafine zinc oxide whose average primary particle size is 10 nm is applied, a powder foundation is applied on top, and then light is irradiated from multiple light sources, sometimes the color of the powder foundation may change significantly.

In the above case, selecting an appropriate pigment for foundation using each of the aforementioned evaluation methods, where such pigment reduces the color change of transmitted light and shade, has the effect of reducing the change in foundation color regardless of the number of light sources. If such measure is not taken, the color changes significantly. Under multiple light sources, the color tends to change to the reddish to yellowish range, which often makes the skin look dull. Under the present invention, each of the aforementioned evaluation methods can also be used to evaluate a coating film comprising multiple pigments. In this case, a coating film subject to less color change and offering excellent UV protection effect can be achieved by, for example, combining a ultrafine titanium dioxide whose average primary particle size is in a range of 1 to 20 nm, a ultrafine zinc oxide, and a large particle size titanium dioxide whose average primary particle size is in a range of 0.4 to 5 µm.

In addition, it has been shown from the results of evaluating pigments based on each of the aforementioned evaluation methods, that although favorable pigments and titanium dioxides vary depending on each evaluation method, large particle size titanium dioxides whose particle size is in a range of 0.4 to 5 µm are favorable because they provide beautiful colors regardless of the number of light sources. Accordingly, large particle size titanium dioxides are explained in details.

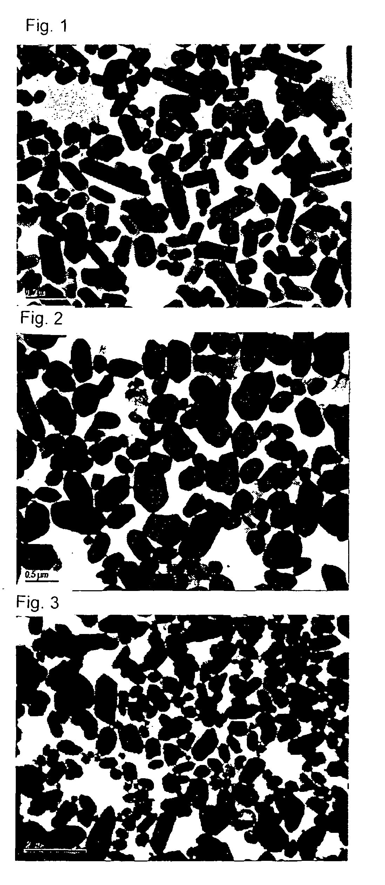

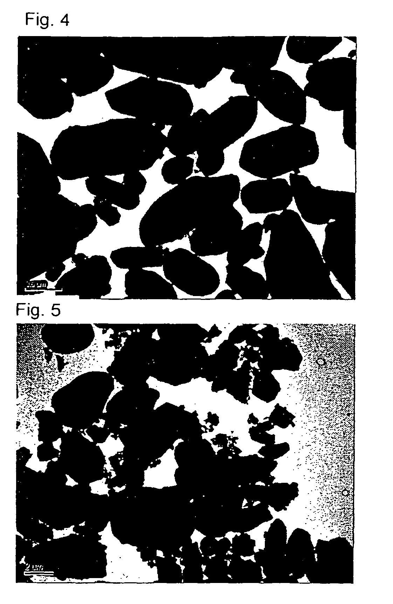

As shown in the transmitting electron micrographs in Figs. 1 through 5, titanium dioxide particles with an average primary particle size of 0.25, 0.5, 0.7, 1.0 and 5.0 µm, respectively, were prepared, and the optical characteristics of clear resin films coated with these titanium dioxide particles were examined according to the second evaluation method. As a result, all samples did not exhibit color, except for the 0.25-µm sample that slightly color appearance, when each sample was irradiated with an incandescent-lamp light source and the transmitted light was irradiated with a fluorescent lamp.

When a yellow lamp was used instead of the incandescent lamp, the transmitted light exhibited red color and the redness increased as the average primary particle size decreased. The concealing property of the pigment had the opposite trend. In general, titanium dioxides are used as concealing agents. To use a titanium dioxide with a large primary particle size, therefore, the content must be increased or a pigment-grade titanium dioxide (corresponding to the 0.25-µm sample used in this example) must be combined to adjust the content. In this case, this evaluation method can be used again to determine an appropriate composition of the formulation by considering the concealing effect, color of shade, and transmitted color. If a pigment-grade titanium dioxide is combined with a large particle size titanium dioxide, favorable benefits can be achieved compared to when a pigment-grade titanium dioxide is used alone, such as providing a coating film having a translucent feel, smooth touch and good spreading property, in addition to favorable optical effects under multiple light sources.

The following explains the present invention in details using examples. It should be noted, however, that the present invention is not at all limited to these examples.

### Examples

### [Example 1]

### Creation of pigment film

Pigment-grade titanium dioxides (JR-405 and JR-800 manufactured by Tayca), ultrafine titanium dioxides (MT-500T and MT-01 manufactured by Tayca), and ultrafine zinc oxide (MZ-500), were each mixed with and dispersed into a polyester resin hexane mixture solution so that the content of each ingredient became 10 percent by weight. A paint shaker was used to disperse the particles. Next, an applicator was used to uniformly coat the obtained dispersion onto a polyethylene terephthalate resin film, after which the film was dried. The coating thickness was set to 10 µm after drying, and the film thickness was checked using a micrometer.

### Light irradiation method:

A color filter (red, green, yellow or blue) was set in front of a 60-W reflector lamp to be used as the first light source. A high-illumination three-wavelength fluorescent lamp (daylight type) was used as the second light source. A pigment film was set in front of the first light source to create shade, and the shade was irradiated with the second light source from diagonally above at the back (from the back at a 20-degree angle to the vertical line of shade). The test was conducted in a dark room.

The test results are shown in Tables 1 and 2.

**[Table 1]**

| When the first light source was used alone | | | | |
|---|---|---|---|---|
| Pigment | Color of shade | | | |
| | Red light | Green light | Yellow light | Blue light |
| JR-405 | Black | Black | Black | Black |
| JR-800 | Black | Black | Black | Black |
| MT-500T | Black | Black | Black | Black |
| MT-01 | Black | Black | Black | Black |
| MZ-500 | Black | Black | Black | Black |

**[Table 2]**

| When the second light source was used in addition to the first light source | | | | |
|---|---|---|---|---|
| Pigment | Color of shade | | | |
| | Red light | Green light | Yellow light | Blue light |
| JR-405 | Blue | Blue | Blue | Blue |
| JR-800 | Blue | Blue | Blue | Blue |
| MT-500T | Blue | Blue | Purple | Blue |
| MT-01 | Light blue | Light blue | Light purple | Light blue |
| MZ-500 | Light blue | Light blue | Light purple | Light blue |

From the above results, MT-01 and MZ-500 that produced relatively less color can be selected as candidate pigments for cosmetics, for example.

### [Example 2]

### Daytime beauty essence:

A daytime beauty essence was created based on the recipe specified in Table 3 and the production method described below. All quantities are indicated in percent by weight. Tospearl 145A manufactured by GE Toshiba Silicones was used as the polymethyl silsesquioxane.

**[Table 3]**

| Ingredient | Content |
|---|---|
| Ethanol | 10 |
| Octyl p-methoxy cinnamate | 5 |
| Polyether denatured silicone | 0.5 |
| Sorbitan monoisostearate | 1 |
| Cyclic pentamer silicone | 28 |
| Crosslinking silicone | 1 |
| Methyl phenyl polysiloxane | 2 |
| Dipropylene glycol | 3 |
| Polymethyl silsesquioxane | 1 |
| Crushed spherical silicone elastomer powder | 1 |
| Silicone-treated ultrafine titanium dioxide MT-01 | 1 |
| Silicone-treated ultrafine zinc oxide MZ-500 | 6 |
| Purified water | Remainder |
| Preservative | As appropriate |

### Production method:

All oil ingredients except for ethanol were mixed, and powder ingredients were added to the mixture and dispersed using a disper, after which ethanol was added and dispersed well. Next, heated water ingredients were added under agitation and the mixture was cooled while all ingredients were mixed well. The cooled mixture was filled into a container to obtain a product.

### [Comparative Example 1]

A product was obtained in the same manner as in Example 1, except that silicone-treated ultrafine titanium dioxide MT-01 was changed to silicone-treated ultrafine titanium dioxide JR-405 among the pigments used in Example 2.

The products obtained by Example 2 and Comparative Example 1 were observed all day based on the skin colors of four subjects under multiple light sources. As a result, the product obtained by Example 2 provided a healthier skin color, less dull complexion and less conspicuous texture irregularity compared to the product obtained by Comparative Example 1.

### [Example 3]

### Production of titanium-dioxide coated film:

Pigment-grade titanium dioxides (JR-405 and JR-800 manufactured by Tayca, having primary particle sizes of 0.21 µm and 0.27 µm, respectively), large particle size titanium dioxide (MPY-100S manufactured by Tayca, having a primary particle size of 1.0 µm), and ultrafine titanium dioxide (MT-500T manufactured by Tayca, having a primary particle size of 35 nm), were each mixed with and dispersed into a polyester resin hexane mixture solution so that the content of each ingredient became 10 percent by weight. A paint shaker was used to disperse the particles. Next, an applicator was used to uniformly coat the obtained dispersion onto a polyethylene terephthalate resin film, after which the film was dried. The coating thickness was set to 2 µm after drying, and the film thickness was checked using a micrometer.

### Light irradiation method:

Using a yellow lamp manufactured by Toshiba Lightec (MUSI100V60WA, with blue light of 450 nm or less cut off) as the first light source, the aforementioned titanium-dioxide coated film was placed 20 cm away from the lamp, and the top of the film was tilted by 30 degrees toward the lamp. Then, the film was irradiated by the second light source, or a 32-W compact fluorescent lamp (daylight color), from a position 40 cm above the irradiating direction of the first light source at an upward angle of 30 degrees as viewed from the coated film.

Each of the titanium dioxides prepared above was measured. As a result, all titanium dioxides showed some reddishness. The degrees of reddishness were: JR-800 > JR-405 >> MPY-100S > MT-500T.
In this evaluation, images captured by a digital camera were observed and compared.

### [Comparative Example 2]

A test was conducted according to Example 3, except that the second light source was turned off and only the first light source was used. As a result, all titanium-dioxide coated films showed yellowish color.

### [Example 4]

Since the results of Example 3 found that, as titanium dioxides having concealing effect, large particle size titanium dioxides would produce less reddishness under multiple light sources, a large particle size titanium dioxide was used to produce a sample powder foundation that also contained cellulose powder and spherical silicone elastomer powder as clear pigments, according to the recipe specified in Table 4 and the production method explained below.
Unless otherwise specified, the pigments used in this example were given surface treatment using N-lauroyl-L-lysine at 5 percent by weight. All quantities are indicated in percent by weight.

**[Table 4]**

| Ingredient | Content |
|---|---|
| Surface-treated large particle size titanium dioxide | 13 |
| Surface-treated cellulose powder | 30 |
| Crushed spherical silicone elastomer powder | 6 |
| Surface-treated sheet-shaped barium sulfate | Remainder |
| Surface-treated iron oxide (yellow, red, black) | 1.5 |
| Silicone-treated ultrafine titanium dioxide | 3 |
| Surface-treated talc | 6 |
| Alkyl denatured silicone wax | 3 |
| Octyl p-methoxy cinnamate | 1 |
| Methyl phenyl polysiloxane | 1 |
| Isononyl isononate | 2 |
| Dimethicone | 3 |
| Liquid paraffin | 1 |
| Preservative | As appropriate |

### Production method:

A silicone-treated ultrafine titanium dioxide was mixed well with a surface-treated talc using a mixer. A surface-treated large particle size titanium dioxide and surface-treated iron oxide were added to the mixture and the ingredients were mixed well to obtain mixture A. The remaining pigments were mixed well using a mixer, and mixture A prepared earlier was added under agitation to mix all ingredients well. While mixing, oil ingredients that had been heated and dissolved were added slowly. Next, the obtained material was crushed, filtered through a mesh, after then stamped using a metal die to obtain a product.

### [Comparative Example 3]

A product was obtained in exactly the same manner as in Example 4, except that JR-800 that exhibited the strongest red in the earlier evaluation was given surface treatment and used instead of the surface-treated large particle size titanium dioxide used in Example 4.

### Evaluation of cosmetics:

The subjects were asked to apply the foundations created by Example 4 and Comparative Example 3 on their faces and the colors of the subject's faces were observed in a room receiving light from the evening sun where both incandescent and fluorescent lamps were also turned on. As a result, depending on the location of observation the foundation obtained by Example 4 looked less reddish, created a more natural color, revealed less texture irregularity, and gave a better impression in the eyes of a third party, compared to the foundation obtained by Comparative Example 3.

### [Example 5]

The image data of the examples and comparative examples were combined to create a document showing the degrees of change in transmitted color under multiple light sources. This document was designed as an at-a-glance guide of color change to allow the viewer to understand the color changes very easily.

### [Example 6]

Films were created in exactly the same manner as in Example 3, except that a commercial shrink film was used instead of the polyethylene terephthalate resin film used in Example 3. Each obtained film was wrapped around a resin bottle containing a lotion, and the film was adhered to the bottle surface using a heat gun. Light was then irradiated from the top right of the container at an angle of 30 degrees using a 60-W incandescent lamp, while another light was irradiated at an upward angle of 60 degrees from a daylight fluorescent lamp positioned in front. As a result, the samples using JR-800 and JR-405 showed high whiteness and a beautiful white appearance when only the fluorescent lamp or incandescent lamp was turned on. Under multiple light sources, however, the container looked murky and its aesthetic appeal decreased considerably. On the other hand, the sample using MPY-100S maintained a beautiful color under both a single light source and multiple light sources. The sample using MT-500T did not provide enough concealing effect and was not suitable as an exterior film for container.

### [Example 7]

Using pigment-grade titanium dioxides (JR-405 and JR-800 manufactured by Tayca, having primary particle sizes of 0.21 µm and 0.27 µm, respectively) and large particle size titanium dioxide (MPY-100S manufactured by Tayca, having a primary particle size of 1.0 µm), each pigment was applied onto the inner part of the front arm of a person using a foundation sponge. As the first light source, a yellow lamp manufactured by Toshiba Lightec (MUSI100V60WA, with blue light of 450 nm or less cut off) was set 30 cm from the arm at an upward angle of 45 degrees. As the second light source, a daylight-color fluorescent lamp manufactured by NEC (330 W; set 2 m to the rear at 45 degrees above the line connecting the first and second light sources) was used to irradiate the arm to the extent that the shade created by the first light source remained sufficiently. The skin condition was observed both visually and using images captured with a digital camera.
As a result, while the skin condition such as unevenness and texture could be observed in details with both pigment-grade titanium dioxides, the skin condition could not be observed clearly, and therefore information could not be obtained regarding the skin surface condition, with the large particle size titanium dioxide.
These results suggest that the large particle size titanium dioxide tested above provides excellent effect in obscuring the skin condition under multiple light sources. On the other hand, the pigment-grade titanium dioxides tested above clearly revealed the skin condition and made the skin problems and roughness more conspicuous under multiple light sources.

### [Example 8]

Red, yellow, green and blue colored lights were obtained by setting color filters in front of a 60-W reflector lamp, used instead of the yellow lamp by Toshiba Lightec used as the first light source in Example 7. A test was conducted using these colored lights according to Example 7. The difference between results with and without the second light source was also observed.
As a result, under red light, little information was obtained regarding the skin condition with any of the titanium dioxides tested when only the first light source was used. Under yellow light and green light, the pigment-grade titanium dioxides allowed the skin condition to be observed visually, but clear observation was not possible with a camera. Under blue light, a difference between the pigment-grade titanium dioxides and large particle size titanium dioxide could be recognized visually, but it was not clear with a camera.
Next, when checked under multiple light sources the pigment-grade titanium dioxides revealed the skin surface condition more clearly under red light. Under yellow light, while the pigment-grade titanium dioxides clearly revealed the skin surface condition, the large particle size titanium dioxide revealed little about the skin condition. Under green light, the pigment-grade titanium dioxides not only revealed the skin condition, but they also made the skin look unsightly to the naked eyes. On the other hand, the large particle size titanium dioxide revealed little about the skin condition. Under blue light, all samples looked whitish, where the level of whiteness was stronger with the pigment-grade titanium dioxides. From the above results, it was found that yellow light and green light could be affecting the human vision in many ways. The results also suggest that the large particle size titanium dioxide tested above is less vulnerable to the effects of light color under multiple light sources compared to the pigment-grade titanium dioxides.

### [Example 9]

Based on the results obtained in Example 8, a digital camera was used to capture how the skin looked differently when large particle size and pigment-grade titanium dioxides were used under a single light source and multiple light sources, and the obtained photographs were attached to a sheet of paper to create a sample display material showing the different appearances of titanium dioxides.

### [Example 10]

Based on the results obtained in Example 8, a cosmetic (concealer) containing a pigment-grade titanium dioxide (JR-405 manufactured by Tayca) and one containing a large particle size titanium dioxide (MPY-100S manufactured by Tayca) were created, and how they looked on the face was evaluated according to the method explained in Example 8. The recipe of the cosmetics is shown in Table 5. The quantities in the table are indicated in percent by weight, while each pigment was octyl-sililated. A non particle type paste was used as the crosslinking silicone paste.

**[Table 5]**

| Ingredient | Content |
|---|---|
| Titanium dioxide | 25 |
| Crosslinking silicone paste | 30 |
| Methyl phenyl polysiloxane | 10 |
| Cyclic pentamer silicone | Remainder |
| Trimethyl siloxysilicate | 5 |
| Dimethicone gum | 2 |

### Production method

The ingredients were mixed using a mixer and the obtained mixture was filled into a bottle to obtain a product.

### Evaluation result

The concealer containing the pigment-grade titanium dioxide revealed irregular skin texture and lines more conspicuously and also made the skin color look dull. On the other hand, the concealer containing the large particle size titanium dioxide did not provide much information regarding the skin and therefore irregular skin texture and lines were not as conspicuous as when the pigment-grade titanium dioxide concealer was applied. When the large particle size titanium dioxide concealer was applied, the skin color did not look dull, either.

## Claims

1. A method for selecting an appropriate pigment when selecting a pigment to be used, comprising utilizing optical characteristics of the pigment obtained when visible light is irradiated onto a target object from two or more light sources.

2. The method for selecting a pigment according to Claim 1, **characterized in that** the pigment is selected by irradiating a first visible light onto a clear resin film coated with a coating solution in which the pigment is dispersed in a clear resin, thereby creating a shade, irradiating a second visible light onto the created shade to the extent not erasing the shade, and then examining a color of a new shade created.

3. The method for selecting a pigment according to Claim 1 or 2, **characterized in that** the clear resin film coated with the pigment is constituted by uniformly coating and drying on the clear resin film a coating solution in which the pigment is dispersed in the clear resin at a content of 10 percent by weight, so that a film thickness becomes 10 µm after drying.

4. The method for selecting a pigment according to any one of Claims 1 to 3, **characterized in that** the first visible light to be irradiated onto the clear resin film coated with the coating solution in which the pigment is dispersed in the clear resin, is yellow light.

5. The method for selecting a pigment according to any one of Claims 1 to 4, **characterized in that** the second visible light is emitted from a fluorescent lamp with a color temperature of 3200K or above.

6. The method for selecting a pigment according to any one of Claims 1 to 5, **characterized in that**, when the color of the shade created by irradiating the second visible light is bluish to purplish, a pigment having less color in this range is selected.

7. A method for selecting a pigment, **characterized in that** selection of the pigment according to any one of Claims 1 to 6 relates to selection of a pigment used in various products.

8. The method for selecting a pigment according to Claim 7, **characterized in that** the pigment is an inorganic powder, organic powder, organic pigment, metal powder for surface active agent, colored pigment, pearl pigment, metal powder pigment, tar dye, or natural dye.

9. The method for selecting a pigment according to Claim 7 or 8, **characterized in that** the pigment is a titanium dioxide.

10. A method for selecting a pigment, **characterized in that** the various products according to Claim 7 are selected from among cosmetics, moldings, containers, paints, exterior wall materials, wall papers, and brochures.

11. The method for selecting a titanium dioxide according to Claim 9, **characterized in that**, when the titanium dioxide is used as the pigment, yellow light is irradiated as a first visible light onto a clear resin film coated with a coating solution in which the titanium dioxide is dispersed in a clear resin, after which a second visible light is irradiated from the side opposite to the one irradiated by the yellow light, and then the titanium dioxide that produces less reddish color is selected based on a difference in red levels as recognized when transmitted yellow light is observed.

12. The method for selecting a titanium dioxide according to Claim 9, **characterized in that**, when the titanium dioxide is used as the pigment, a clear resin film is uniformly coated with a coating solution in which the titanium dioxide is dispersed in a clear resin at a content of 10 percent by weight, and then the coating solution is dried to create a film of 2 µm in thickness after drying.

13. The method for selecting a titanium dioxide according to Claim 9, **characterized in that**, when the titanium dioxide is used as the pigment, the visible light irradiated onto a clear resin film coated with a coating solution in which the titanium dioxide is dispersed in a clear resin is emitted from a fluorescent lamp with a color temperature of 3200K or above.

14. The method for selecting a titanium dioxide according to Claim 9, **characterized in that** a first visible light is irradiated onto an object coated with a coating solution in which the titanium dioxide is dispersed in a clear resin, after which a second visible light is irradiated onto the object from a different light source, and then the titanium dioxide that makes the surface condition of the object less conspicuous is selected.

15. The method for selecting a titanium dioxide according to Claim 9, **characterized in that** a first visible light is irradiated onto an object, after which a second visible light is irradiated onto the object from a different light source and the appearance of the titanium dioxide is captured with a camera, and then inconspicuousness of a surface condition of the object is evaluated by comparing the appearance of the surface.

16. The method for selecting a titanium dioxide according to Claim 9, **characterized in that** a first visible light has a color other than red.

17. The method for selecting a titanium dioxide according to Claim 9, **characterized in that** a second visible light is light from a fluorescent lamp.

18. The method for selecting a titanium dioxide according to Claim 9, **characterized in that** a primary particle size of the titanium dioxide that makes a surface condition of the object inconspicuous is not in a range of 0.1 µm or larger and smaller than 0.4 µm, but in a range of 0.05 µm or larger and smaller than 0.1 µm, or in a range of 0.4 µm or larger and 5 µm or smaller.

19. The method for selecting a titanium dioxide according to Claim 9, **characterized in that** the object is selected from among human skin, synthetic leather and leather.

20. A cosmetic **characterized by** having a beautiful color that is retained even under two or more light sources, and containing an organic pigment or clear pigment as well as a pigment selected by irradiating a first visible light onto a clear resin film coated with a coating solution in which a pigment selected according to any one of Claims 1 to 9 is dispersed in a clear resin, and then irradiating a second visible light onto the created shade to the extent not erasing the shade, after which a color of the shade created by the second visible light is examined and a pigment having less color is selected.

21. The cosmetic according to Claim 14, **characterized in that** a total content of one or more titanium dioxides is in a range of 1 to 15 percent by weight relative to the total weight of the cosmetic, while a total content of one or more clear pigments is in a range of 0.1 to 80 percent by weight relative to the total weight of the cosmetic.

22. A molding **characterized by** having a coating film showing a beautiful color, which is achieved by a color of the shade formed by irradiating a first visible light onto a clear resin film coated with a coating solution in which a pigment selected according to any one of Claims 1 to 9 is dispersed in a clear resin, and then irradiating a second visible light onto the created shade to the extent not erasing the shade.

23. A molding **characterized by** use of a less bluish titanium dioxide, which is obtained by evaluating a color of the shade formed by irradiating a first visible light onto a film coated with a titanium dioxide, which is used as a pigment and selected according to any one of Claims 1 to 9, and then irradiating a second visible light onto the created shade to the extent not erasing the shade.

24. A display material showing an evaluation image of an item containing a titanium dioxide selected according to any one of Claims 1 to 9.
